Europäisches Patentamt

(19) **European Patent Office**

**Office européen des brevets**

(11) Veröffentlichungsnummer: **0 025 580**
A1

(12) # EUROPÄISCHE PATENTANMELDUNG

(21) Anmeldenummer: **80105376.0**

(22) Anmeldetag: **09.09.80**

(51) Int. Cl.³: **C 07 G 7/00,** A 23 J 3/00,
C 12 N 1/06

(30) Priorität: **18.09.79 DE 2937616**

(43) Veröffentlichungstag der Anmeldung: **25.03.81**
**Patentblatt 81/12**

(84) Benannte Vertragsstaaten: **AT BE CH DE FR GB IT LI LU
NL SE**

(71) Anmelder: **BASF Aktiengesellschaft,
Carl-Bosch-Strasse 38, D-6700 Ludwigshafen (DE)**

(72) Erfinder: **Bonitz, Eckhard, Dr., Carostrasse 23,
D-6710 Frankenthal (DE)**
Erfinder: **Buckel, Klaus, Erzbergerstrasse 74,
D-6700 Ludwigshafen (DE)**
Erfinder: **Daucher, Hans, Dr., Dubliner Strasse 23,
D-6700 Ludwigshafen (DE)**
Erfinder: **Klink, Hans, Riedstrasse 33, D-6711 Hessheim
(DE)**
Erfinder: **Stickel, Richard, Auf dem Koeppel, D-6702 Bad
Duerkheim 1 (DE)**

(54) Verfahren zur Herstellung von Hydrolysevorprodukten, Hydrolyseprodukten und Aminosäuren aus Proteinen oder Proteine enthaltenden Stoffen.

㊗ Behandlung mittels Chlorwasserstoffsäure, wobei man auf die im wesentlichen wasserfreie Proteine oder Proteine enthaltende Stoffe Chlorwasserstoffgas einwirken lässt.

Verfahren zur Herstellung von Hydrolysevorprodukten,
Hydrolyseprodukten und Aminosäuren aus Proteinen oder
Proteine enthaltenden Stoffen

Seit langem ist bekannt, Proteine mit Hilfe von wäßriger Chlorwasserstoffsäure zu hydrolysieren. Als Produkte erhält man bei vollständiger Hydrolyse Aminosäuren. Im Falle teilweiser Hydrolyse erhält man niedere oligomere Peptide neben freien Aminosäuren.

Zur Durchführung von Hydrolysen ist erfahrungsgemäß eine verhältnismäßig große Flüssigkeitsmenge erforderlich, die in Form von wäßriger Salzsäure angewandt wird und die zugleich das flüssige Dispersionsmedium darstellt. Der dabei angewandte Überschuß an wäßriger Säure muß zur Gewinnung der Reaktionsprodukte durch Eindampfen unter vermindertem Druck entfernt werden. Diese Verfahrensweise erfordert einen hohen Energieaufwand, einmal durch das Erfordernis langer Aufschlußzeiten bei Siedetemperatur, zum anderen, weil die Entfernung der Säure mit einer hohen Verdampfungsleistung für das azeotrop entweichende Wasser verbunden ist.

Es bestand daher die Aufgabe, den Energiebedarf der Hydrolysereaktion zu verringern und die Nebenproduktbildung wie z.B. Racemisierung der Aminosäuren herabzusetzen.

Hp/BL

0025580

Diese Aufgabe wurde erfindungsgemäß mit einem Verfahren zur Herstellung von Hydrolysevorprodukten, Hydrolyseprodukten und Aminosäuren aus Proteinen oder Proteine enthaltenden Stoffen durch Behandlung mittels Chlorwasserstoffsäure dadurch gelöst, daß man auf im wesentlichen wasserfreie Proteine oder Protein enthaltende Stoffe Chlorwasserstoffgas einwirken läßt.

Unter im wesentlichen wasserfreien Proteinen werden solche mit einem Feuchtigkeitsgehalt unter 20 Gew.%, vorzugsweise unter 10 Gew.% verstanden. Bei der Einwirkung von gasförmigem Chlorwasserstoff bei normalem Druck oder leicht erhöhtem Druck (bis 10 bar, z.B. bei schwerhydrolysierbaren Proteinen wie Haaren oder Horn), wird eine begrenzte Menge Chlorwasserstoff unter Wärmeentwicklung aufgenommen.

Die Einwirkung von Chlorwasserstoffgas kann bis zur Sättigung geführt werden. In der Regel genügen jedoch geringere Mengen. Damit wasserlösliche Produkte erhalten werden, wird man aber solange die Umsetzung mit Chlorwasserstoff fortführen bis eine solche Menge aufgenommen worden ist, daß das Reaktionsprodukt beim Anpasten mit

1 bis 4M HCl

1 bis 4M $H_2SO_4$

1 bis 4M NaOH

und Stehenlassen bei erhöhter Temperatur, z.B. bei 60° bis 95°C ein bezogen auf den Proteinanteil vollständig wasserlösliches Hydrolysat liefert. Diese Wasserlöslichkeit der Hydrolyseprodukte wird teils im sauren, teils im neutralen und teils im basischen Bereich gefunden.

Bei der erfindungsgemäßen Behandlung mit Chlorwasserstoffgas tritt eine für das jeweilige Produkt charakteristische Färbung der trockenen Masse auf. Bei bestimmten Einzellerproteinen wird z.B. eine intensive Rotfärbung beobachtet.

0025580

Diese Produkte stellen vermutlich Anlagerungsverbindungen nach Art von Hydrochloriden von Proteinen dar. Sie können unmittelbar als sterile Ausgangsprodukte für Futtermittelzusatzstoffe in den Handel gebracht werden und gehen mit dem Wassergehalt der Futtermittel, denen sie zugesetzt werden, allmählich in lösliche Produkte über.

In der Regel werden aber aus den Anlagerungsprodukten mit Wasser, vorzugsweise in der Wärme, Polypeptide, Oligopeptide und Aminosäuren unmittelbar freigesetzt und nach bekannten Methoden isoliert.

Beispielsweise werden die trockenen Anlagerungsprodukte mit wenig Wasser zu Pasten verrührt und diese z.B. bei erhöhter Temperatur bis zu 100°C eine gewisse Zeit aufbewahrt; sie sind dann in lösliche Produkte übergegangen.

Falls die Proteine Begleitstoffe enthalten, die bei Wassereinwirkung Säure verbrauchen (z.B. Kalk bei Fischmehl), wird in der zweiten Stufe anstelle von Wasser zweckmäßig eine verdünnte Säure zugesetzt.

Gegebenenfalls nach Neutralisation können die löslichen Hydrolyseprodukte noch mit Fermenten (Enzymen) behandelt werden, um vollständig oder nahezu vollständig hydrolysierte Endprodukte insbesondere freie Aminosäuren freizusetzen.

Im einzelnen wird das erfindungsgemäße Verfahren z.B. folgendermaßen ausführt: In einem Behälter mit Rührvorrichtung oder in einer Wirbelschicht wird ein pulverförmiges Protein mit gasförmigem Chlowasserstoff umgesetzt, wobei die sich entwickelnde Wärme durch Kühlen der Behälterwände oder mit Hilfe eines inerten Gases wie Stickstoff abgeführt wird. Im Verlauf von 30 bis 120 min wird

0025580

lebhaft Chlorwasserstoffgas aufgenommen. Bei Unterbrechung der Chlorwasserstoffzufuhr vor der Sättigung, tritt Druckverminderung im Reaktionsraum ein. Das Produkt nimmt erfahrungsgemäß zwischen 2 bis 20 % seiner Masse bezogen auf das Endgewicht Chlorwasserstoff auf. Man erhält ein rieselfähiges Produkt (1), das an der Luft leicht raucht und nach Chlorwasserstoff riecht.

Gibt man dieses Produkte (1) in einen Raum mit einem definierten Wasserdampfpartialdruck, z.B. 0,033 bar so wird Wasser aufgenommen. Nach beendeter Wasseraufnahme ist das Produkt (2) ebenfalls rieselfähig. Wird ein solches Produkt (2) gelagert z.B. bei Umgebungstemperatur, so wird es allmählich hydrolysiert. Das erkennt man beispielsweise daran, daß ein ursprünglich wasserunlösliches Produkt auch ohne Zufuhr von Wärme allmählich wasserlöslich wird.

Die erfindungsgemäße Umsetzung von Proteinen nacheinander mit Chlorwasserstoffgas und anschließender Hydrolyse kann aber auch gleichzeitig mit gasförmigen Gemischen aus Chlorwasserstoffgas und Wasserdampf erfolgen. Dabei werden Hydrolyseprodukte der Proteine unmittelbar erhalten.

Die Behandlung verschiedener Proteine zeigt folgende Tabelle:

| | Protein enthaltendes Ausgangsmaterial | Rest- wasser | | Chlorwasser- stoffaufnahme | | Färbungen vom | |
|---|---|---|---|---|---|---|---|
| | | | | | | Ausgangs- produkt | Chlorwasser- stoffaddukt |
| 1 | Biotrockenmasse Fermenterprodukt von Caprolactam- abwasser | 7 | % | 9,4 % | | hellbeige | violett-rot |
| 2 | Biotrockenmasse Klärschlamm | 8 | % | 12,0 % | | hellbraun | kakaobraun |
| 3 | Fischmehl nicht entfettet | 9 | % | 9 % | | hellbraun | dunkelbraun |
| 4 | Fischmehl entfettet | 1 | % | 17,0 % | | hellbraun | dunkelbraun |
| 5 | Sojaschrot entfettet | 10,5 % | | 16,3 % | | hellbeige | dunkelbraun |
| 6 | Schlachterei- abfälle ent- fettet | 6 | % | 10,5 % | | braun | dunkelbraun |
| 7 | Biotrockenmasse Fermenterprodukt von Propylenoxid- abwasser | 2,5 % | | 9,0 % | | grau | braun |
| 8 | Gelatine | 10,9 % | | 8,0 % | | gelb | gelbbraun |
| 9 | Milchpulver 34 % Proteine | 5,9 % | | 8,0 % | | gelblich weiß | altrosa |

| Protein enthaltendes Ausgangsmaterial | Rest-wasser | Chlorwasser-stoffaufnahme | Färbungen vom | |
|---|---|---|---|---|
| | | | Ausgangs-produkt | Chlorwasser-stoffaddukt |
| Casein aus Milch 80 % Proteine | 9,2 % | 17,0 % | gelblich weiß | altrosa |
| Bäckerhefe | 12,3 % | 17,7 % | grauweiß | violett-rot |

## Beispiel 1 Bäckerhefe

### 1.1 Trocknung

1000 Teile frische Bäckerhefe werden in 1600 Teilen Methanol dispergiert, abfiltriert und bei 50°C getrocknet. Dadurch werden schmierige Bestandteile entfernt. Es werden 260 Teile eines feinen, grauweißen Pulvers mit einer Partikelgröße von <200 um erhalten.

### 1.2 Herstellung des HCl-Adduktes

100 Teile Trockenhefe mit einem Restwassergehalt von 12,3 Gew.% werden unter Rühren mit Chlorwasserstoff-Gas behandelt, indem man dieses derart feindosiert über das Pulver leitet, daß es sich nicht über 50°C erwärmt. Dieser Vorgang dauert 1 bis 1,5 Stunden. Es wird ein violett-rotes Pulver erhalten, das bezogen auf das Endgewicht 17,7% HCl aufgenommen hat.

### 1.3 Hydrolyse mit Wasser unter Druck

100 Teile des violett-roten HCl-Adduktes werden mit 50 Teilen heißem Wasser angepastet. Die Masse nimmt dabei eine braune Färbung an. Dann erwärmt man diese 10 Stunden in einem geschlossenen Gefäß auf 95°C. Dabei wird die Masse dünnerflüssig. Danach extrahiert man mit 500 Teilen heißem Wasser. Die filtrierte dunkelbraune Lösung wird in einem Verdampfer unter vermindertem Druck bei 60°C eingedampft. Der Trockenrückstand beträgt 64 Teile. Er besitzt einen angenehmen würzigen Geruch und Geschmack und besteht zu etwa 45 Gew.% aus Aminosäuren. Im Verlauf dieser Umsetzung werden von 100 Teilen des violetten HCl-Adduktes 92 % in wasserlösliche Form übergeführt. Es bleibt ein Rückstand von 8%, der in 4 MNaOH löslich ist.

1.4 Man verfährt wie in Beispiel 1.3 und pastet 100 Teile des HCl-Adduktes mit 100 Teilen 1 MHCl an. In einem Gefäß mit Rührer und Rückflußkühler, der gegen die Atmosphäre offen ist, erwärmt man die Masse unter Rühren 20 Stunden auf 95°C. Dabei wird sie dünnflüssig. Man löst das dunkelbraune Reaktionsprodukt in 500 Teilen Wasser und filtriert vom Rückstand (8%) ab. Der Trockenrückstand aus der wässrigen Lösung besteht zu 55% aus Aminosäuren.

1.5 Hydrolyse mit 4 MNaOH   .

Man verfährt wie in Beispiel 1.3 und pastet 100 Teile des HCl-Adduktes mit 100 Raumteilen 4 MNaOH an.

Dabei erhitzt sich die Masse, wird dünnflüssig und dunkelbraun. Sie ist in 800 Teilen Wasser vollständig löslich und zeigt in dieser Lösung einen pH-Wert von 9 bis 10.

Beispiel 2 Biotrockenmasse eines Fermenterproduktes von Abwasser der Caprolactamherstellung

2.1  Trocknung

2000 Teile feuchte Biomasse eines Fermenterproduktes vom Abwasser der Caprolactamherstellung werden in 2000 Teilen Aceton dispergiert und zentrifugiert. Das Sediment wird unter vermindertem Druck 25 Torr $\stackrel{\wedge}{=}$ 33 mbar bei 50°C getrocknet. Man erhält 450 Teile eines hellbeigen Pulvers mit einer Partikelgröße $<100$ µm und einem Restwassergehalt von 7 Gew.%.

0025580

2.2 Herstellung des HCl-Adduktes

Man verfährt wie in Beispiel 1.2 angegeben und erhält ein violett-rotes Produkt, das einen HCl-Gehalt von 9,4 Gew.% aufweist. Dieses Produkt ist hygroskopisch und nimmt beim Lagern an feuchter Luft 15 bis 20 Gew.% Wasser auf, wobei es eine dunkelbraune Färbung annimmt.

2.3 Hydrolyse mit Wasser in der Wärme unter Druck

100 Teile des violett-roten HCl-Adduktes werden mit 100 Teilen heißem Wasser angepastet, wobei die Masse eine dunkelbraune Färbung annimmt. Dann erwärmt man diese 2 Stunden in einem geschlossenen Gefäß auf 80°C. Danach löst man das Reaktionsprodukt in 500 Teilen heißem Wasser und filtriert vom Rückstand (12 Teile) ab. Die Lösung wird mit 4 MNaOH auf pH 5,5 eingestellt und unter vermindertem Druck bei 60°C zur Trockne eingedampft. Von 100 Teilen HCl-Addukt erhält man 75 Gew.% wasserlösliches Hydrolysat, das zu 80% aus Aminosäuren besteht.

2.4 Hydrolyse mit Wasser bei Umgebungstemperatur

Man stellt gemäß 2.2 das HCl-Addukt her, lagert es in feuchter Luft bei einem Wasserdampfpartialdruck von 33 mbar, bis es 15 Gew.% Wasser, bezogen auf das Endgewicht, aufgenommen hat.

Dieses lagert man in einem geschlossenen Raum mit einem HCl-Partialdruck von 50 mbar bei 25°C. Man erhält nach einem Lagern von 2 Wochen ein dunkelbraunes Produkt, das etwa zu 60 Gew.% in heißem Wasser löslich ist.

0025580

Beispiel 3 Fischmehl

3.1 Entfettung und Trocknung

1000 Teile Fischmehl werden in einem Extraktor bei 25°C mit 1500 Teilen Methanol extrahiert. Der Methanolextrakt enthält ein nach Tran riechendes öliges Produkt. Nach dem Trocknen bei 50°C unter vermindertem Druck (25 Torr $\hat{=}$ 33 mbar) erhält man eine krümelige, hellbraune Masse, die auf einer Schlagkreuzmühle zu einem Pulver vermahlen wird. Eine Siebfraktion mit einer Partikelgröße < 200 um zeigte einen Restwassergehalt von 1 Gew.%.

3.2 Herstellung des HCl-Adduktes und Hydrolyse

100 Teile des entfetteten und getrockneten Fischmehls werden auf einen Wirbelboden mit Stickstoff gewirbelt und allmählich mit Chlorwasserstoffgas umgesetzt. Die Aufnahme an HCl-Gas beträgt bezogen auf das Endgewicht 17 Gew.%. Man erhält bei einer Temperatur von 40 bis 50°C ein dunkelbraunes Pulver. Es ist hygroskopisch und nimmt bei einem Wasserdampfpartialdruck von 33 mbar 20 bis 25 Gew.%, bezogen auf das Endgewicht, Wasser auf. Wird dieses feuchte Produkt 20 Stunden in einem geschlossenen Raum bei 90°C gelagert, so ist es anschließend zu 93% in Wasser löslich.

Beispiel 4 Gelatine

4.1 Herstellung des HCl-Adduktes

Man verfährt wie in Beispiel 1.2 und setzt Gelatine (aus Knochen) mit einer Partikelgröße von 200 um und

0025580

einem Wassergehalt von 10,9 Gew.% mit Chlorwasser-stoff-Gas um. Man erhält aus dem gelblichen Gelatine-pulver einen tiefbraunen Stoff, der einen HCl-Gehalt von 8,0 Gew.%, bezogen auf das Endgewicht, aufweist.

4.2 Hydrolyse mit 1 MHCl unter Atmosphärendruck

100 Teile des dunkelbraunen HCl-Adduktes werden mit 100 Teilen 1 MHCl angepastet. In einem Gefäß mit Rührer und Rückflußkühler wird die Mischung 15 Stunden bei 95°C gerührt, wobei sie dünnflüssig wird. Das Reaktionsprodukt ist in 500 Teilen Wasser vollständig löslich. Die Lösung bleibt dünnflüssig und besitzt gelbbraune Färbung. Sie enthält neben Peptiden Amino-säuren.

989/21-33

Patentansprüche

1. Verfahren zur Herstellung von Hydrolysevorprodukten, Hydrolyseprodukten und Aminosäuren aus Proteinen oder Proteine enthaltenden Stoffen durch Behandlung mittels Chlorwasserstoffsäure, dadurch gekennzeichnet, daß man auf im wesentlichen wasserfreie Proteine oder Proteine enthaltende Stoffe Chlorwasserstoffgas einwirken läßt.

2. Verfahren gemäß Anspruch 1, dadurch gekennzeichnet, daß man die Einwirkung durch Überleiten von Chlorwasserstoffgas über die Proteine oder Proteine enthaltende Stoffe vornimmt.

3. Verfahren gemäß Anspruch 1, dadurch gekennzeichnet, daß man die Proteine oder Proteine enthaltenden Stoffe in der Wirbelschicht mit Chlorwasserstoff behandelt, wobei ein unter den Reaktionsbedingungen inertes Gas als Wirbelgas verwendet wird.

4. Verfahren gemäß Anspruchen 1 bis 3, dadurch gekennzeichnet, daß man Chlorwasserstoffgas bei normaler Temperatur oder nur leicht erhöhter Temperatur unter Abführung der Reaktionswärme einwirken läßt.

5. Verfahren gemäß Ansprüchen 1 bis 4, dadurch gekennzeichnet, daß man Chlorwasserstoffgas bis zur Aufnahme mindestens solcher Mengen einwirken läßt, daß das Reaktionsprodukt nach anschließendem Anpasten mit verdünnter Säure oder Lauge beim Stehen bei erhöhter Temperatur vollständig oder nahezu vollständig in wasserlösliche Hydrolyseprodukte übergeht.

6. Verfahren gemäß Anspruch 1, dadurch gekennzeichnet, daß man die Umsetzungsprodukte mit Wasser, verdünnten Säuren, Basen und/oder Enzymen zur Überführung in wasserlösliche Hydrolyseprodukte behandelt.

Europäisches
Patentamt

**EUROPÄISCHER RECHERCHENBERICHT**

0025580

Nummer der Anmeldung

EP 80 10 5376

| **EINSCHLÄGIGE DOKUMENTE** | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| X | DE - C - 673 203 (DR. W. GRASSMANN)<br>* Seite 1, Zeilen 4-16; Seite 1, Zeile 37 - Seite 2, Zeile 14; Beispiel 2; Ansprüche 1,2 *<br><br>-- | 1,2,5,6 | C 07 G 7/00<br>A 23 J 3/00<br>C 12 N 1/06 |
| A | FR - A - 991 125 (M.M.C. GUILBAUD) | | |
| A | DE - C - 108 053 (DR. H. BREMER)<br><br>---- | | |

RECHERCHIERTE SACHGEBIETE (Int. Cl.³)

C 07 G 7/00
A 23 J 3/00
C 12 N 1/06

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 18-12-1980 | SCHUERMANS |

EPA form 1503.1  06.78